(19) Europäisches Patentamt
European Patent Office
Office européen des brevets

(11) Veröffentlichungsnummer : **0 083 750**
**B1**

(12) # EUROPÄISCHE PATENTSCHRIFT

(45) Veröffentlichungstag der Patentschrift : **07.08.85**

(21) Anmeldenummer : **82111535.9**

(22) Anmeldetag : **13.12.82**

(51) Int. Cl.⁴ : **A 61 K 31/41**, A 61 K 31/415// C07D249/08, C07D233/60, C07D233/54

(54) **Antimykotische Azolyl-butanole.**

(30) Priorität : **24.12.81 DE 3151440**

(43) Veröffentlichungstag der Anmeldung : **20.07.83 Patentblatt 83/29**

(45) Bekanntmachung des Hinweises auf die Patenterteilung : **07.08.85 Patentblatt 85/32**

(84) Benannte Vertragsstaaten : **AT BE CH DE FR GB IT LI NL**

(56) Entgegenhaltungen :
**EP-A- 0 020 955**
**EP-A- 0 055 833**

(73) Patentinhaber : **BAYER AG**
**Konzernverwaltung RP Patentabteilung**
**D-5090 Leverkusen 1 Bayerwerk (DE)**

(72) Erfinder : **Krämer, Wolfgang, Dr.**
**Am Eckbusch 39/45**
**D-5600 Wuppertal (DE)**
Erfinder : **Büchel, Karl Heinz, Prof.Dr.**
**Dabringhausener Strasse 42**
**D-5093 Burcheid (DE)**
Erfinder : **Elbe, Hans-Ludwig, Dr.**
**Dasnoeckel 59**
**D-5600 Wuppertal 11 (DE)**
Erfinder : **Kraatz, Udo, Dr.**
**Andreasstrasse 22a**
**D-5090 Leverkusen (DE)**
Erfinder : **Regel, Erik, Ing.-grad.**
**Untere Bergerheide 26**
**D-5600 Wuppertal 1 (DE)**
Erfinder : **Plempel, Manfred, Dr.**
**Zwengenberger Strasse 3c**
**D-5657 Haan (DE)**
Erfinder : **Schaller, Klaus, Dr.**
**Bergerheide 47**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 083 750 B1

Jouve, 18, rue St-Denis, 75001 Paris, France

**Beschreibung**

Die vorliegende Erfindung betrifft die Verwendung von neuen substituierten 1-Azolyl-butan-2-olen als antimykotische Mittel.

Es ist bereits bekannt geworden, daß bestimmte Phenylazolylmethyl-carbinole, wie z. B. 1-(4-Chlorphenyl)-1-hydroxy-2-(1,2,4-triazol-1-yl)-propan oder 1-(4,4'-Chlorbiphenylyl-1-hydroxy-2-(1,2,4-triazol-1-yl)-ethan, fungizide Eigenschaften besitzen (vgl. DE-OS 24 31 407). Die Wirkung dieser Azolyl-Derivate gegen humapathogene Pilze ist jedoch nicht immer befriedigend.

Es wurde gefunden, daß die neuen substituierten 1-Azolyl-butan-2-ole der allgemeinen Formel

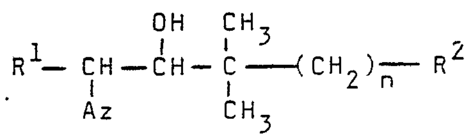

(I)

in welcher

Az für 1,2,4-Triazol-1-yl und -4-yl oder Imidazol-1-yl steht,

n für 0 oder 1 steht,

$R^1$ für geradkettiges oder verzweigtes Alkyl mit 1 bis 6 Kohlenstoffatomen, Alkenyl und Alkinyl mit jeweils 2 bis 6 Kohlenstoffatomen, gegebenenfalls durch Methyl substituiertes Cyclohexyl und Cyclohexylmethyl, sowie für gegebenenfalls substituiertes Phenoxyalkyl und gegebenenfalls substituiertes Phenylalkyl mit jeweils 1 bis 2 Kohlenstoffatomen im Alkylteil steht, wobei als Phenyl-Substituenten genannt seien ; Fluor, Chlor, Methyl, Ethyl, Isopropyl, tert.-Butyl, Dimethylamino, Methoxy, Methylthio, Cyclohexyl, Trifluormethyl, Trifluormethoxy, Trifluormethylthio, Nitro, Cyano sowie gegebenenfalls durch Fluor und Chlor substituiertes Phenyl und Phenoxy, sowie die Morpholinocarbinol-, Phenylaminocarbonyl-, Chlorphenylaminocarbonyl- und Dibutylaminocarbonyl-Gruppe ;

$R^2$ für gegebenenfalls substituiertes Phenyl steht, wobei als Substituenten die bei $R^1$ bereits genannten Phenylsubstituenten infrage kommen, ferner für Cyano, Methoxycarbonyl, Ethoxycarbonyl, Isopropoxycarbonyl sowie die Gruppierungen $—X—R^3$ und $—CO—NR^4R^5$ steht, in welchen

X für Sauerstoff, Schwefel, die SO- oder $SO_2$-Gruppe steht,

$R^3$ für geradkettiges oder verzweigtes Alkyl mit 1 bis 4 Kohlenstoffatomen oder gegebenenfalls substituiertes Phenyl und Benzyl steht, wobei als Substituenten die bei $R^1$ bereits genannten Phenylsubstituenten infrage kommen,

$R^4$ für Wasserstoff, Methyl, Ethyl, Isopropyl oder gegebenenfalls substituiertes Phenyl steht, wobei als Substituenten Halogen und Alkyl mit 1 bis 4 Kohlenstoffatomen infrage kommen ; und

$R^5$ für Wasserstoff, Methyl oder Isopropyl steht,

sowie deren Säureadditionssalze gute antimykotische Eigenschaften aufweisen.

Die Verbindungen der Formel (1) besitzen zwei assymetrische Kohlenstoffatome ; sie können dann in den beiden geometrischen Isomeren (threo- und erythro-Form) vorliegen, die in unterschiedlichen Mengenverhältnissen anfallen können. In beiden Fällen liegen sie als optische Isomeren vor.

Überraschenderweise zeigen die erfindungsgemäß zu verwendenden substituierten 1-Azolyl-butan-2-ole eine bessere antimykotische Wirkung als die aus dem Stand der Technik bekannten Phenylazolylmethyl-carbinole, welche chemisch und wirkungsgemäß naheliegende Verbindungen sind. Die erfindungsgemäße Verwendung der neuen Stoffe stellt somit eine Bereicherung der Pharmazie dar.

Die erfindungsgemäß zu verwendenden Wirkstoffe sind noch nicht bekannt ; sie sind jedoch Gegenstand einer eigenen älteren Patentanmeldung (vgl. EP-A-55 833) und können nach dem dort angegebenen Verfahren erhalten werden, indem man substituierte 1-Azolyl-butan-2-one der Formel

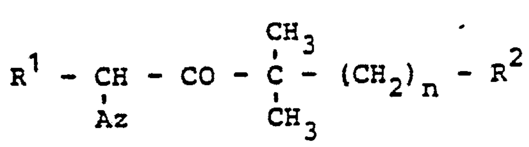

(II)

in welcher Az, $R^1$, $R^2$ und n die oben angegebene Bedeutung haben,

in üblicher Weise reduziert, wie z. B. durch Umsetzung mit komplexen Hydriden, wie Natriumborhydrid oder Lithiumalanat, in Gegenwart eines polaren organischen Lösungsmittels, wie z. B. Alkohole, bei Temperaturen von ca. 0 bis 30 °C ; oder durch Umsetzung mit Aluminiumisopropylat in Gegenwart eines Verdünnungsmittels, wie z. B. Isopropanol, bei Temperaturen von ca. 20 bis 120 °C.

An die so erhaltenen Verbindungen der Formel (I) kann gegebenenfalls anschließend eine Säure addiert werden. In manchen Fällen erweist es sich als vorteilhaft, die Verbindungen der Formel (I) über ihre Salze in reiner Form zu erhalten.

Die substituierten 1-Azolyl-butan-2-one der Formel (II) sind noch nicht bekannt ; sie werden erhalten, indem man

a) Halogenketone der Formel

$$Hal - CH_2 - CO - \overset{CH_3}{\underset{CH_3}{C}} - (CH_2)_n - R^2 \qquad (III)$$

in welcher

Hal für Halogen, insbesondere Chlor oder Brom steht und

$R^2$ und n die oben angegebene Bedeutung haben, wobei jedoch in der Gruppierung —X—$R^3$ der Substituent X nur für Sauerstoff oder Schwefel steht,

mit Azolen der Formel

$$H—Az \qquad (IV)$$

in welcher Az die oben angegebene Bedeutung hat,

in Gegenwart eines inerten organischen Lösungsmittels, wie beispielsweise Acetonitril, und in Gegenwart eines Säurebindemittels, wie beispielsweise Kaliumcarbonat oder ein Überschuß an Azol, bei Temperaturen zwischen 60 und 120 °C umsetzt ; und gegebenenfalls

b) die so erhaltenen Verbindungen der Formel

$$Az - CH_2 - CO - \overset{CH_3}{\underset{CH_3}{C}} - (CH_2)_n - R^2 \qquad (IIa)$$

in welcher Az, n und $R^2$ die oben angegebene Bedeutung haben,

mit einem Alkylierungsmittel der Formel

$$R^1—Z \qquad (V)$$

in welcher

$R^1$ die oben angegebene Bedeutung hat und

Z für eine elektronenanziehende Abgangsgruppe, wie Halogen, p-Methylphenylsulfonyloxy oder Sulfat steht,

in üblicher Weise in Gegenwart eines inerten organischen Lösungsmittels, wie beispielsweise Dimethylformamid, bei Temperaturen zwischen 0 und 100 °C umsetzt und gegebenenfalls

c) die nach den Verfahren (a) und (b) erhaltenen Verbindungen der Formel

$$R^1 - \underset{Az}{CH} - CO - \overset{CH_3}{\underset{CH_3}{C}} - (CH_2)_n - S - R^3 \qquad (IIb)$$

in welcher Az, $R^1$, n und $R^3$ die oben angegebene Bedeutung haben,

nach bekannten Methoden in üblicher Weise oxidiert, wie z. B. durch Umsetzung mit Wasserstoffperoxid in Eisessig bei Temperaturen zwischen − 30 und 80 °C. Bei der Durchführung der Oxidation setzt man auf 1 Mol der Verbindungen der Formel (IIb) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Acetanhydrid bei Temperaturen zwischen − 30 bis + 30 °C, entstehen Verbindungen der Formel (II), bei denen X für SO steht. Bei Überschuß an Oxidationsmitteln und höheren Temperaturen (10 bis 80 °C) entstehen vorzugsweise solche Verbindungen der Formel (II), bei denen X für $SO_2$ steht. Die Isolierung der Oxidationsprodukte erfolgt in üblicher Weise.

Die Halogenketone der Formel (III) sind teilweise bekannt (vergleiche DE-OS 2 635 663). Sie werden erhalten, indem man Ketone der Formel

$$CH_3 - CO - \overset{CH_3}{\underset{CH_3}{C}} - (CH_2)_n - R^2 \qquad (VI)$$

in welcher $R^2$ und n die oben angegebene Bedeutung haben,

mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungsmitteln, wie beispielsweise Sulfurylchlorid, bei 20 bis 60 °C umsetzt.

Die Ketone der Formel (VI) sind teilweise bekannt (vergleiche J. Org. Chem. *42,* 1709-1717 (1977) ; J. Am. Chem. Soc. *98,* 7882-84 (1976) ; J. Org. Chem. *37,* 2834-2840 (1972), US-Patentschrift 3 937 738 sowie C.A. *82,* 20898j (1975)). Sie können erhalten werden, indem man z. B. Keto-Derivate der Formel

$$CH_3 - CO - \underset{CH_3}{\overset{CH_3}{\underset{|}{\overset{|}{C}}}} - (CH_2)_n - Y \qquad (VII)$$

in welcher

n die oben angegebene Bedeutung hat und

Y für Chlor, Brom oder die Gruppierung $-O-SO_2-R^6$ steht, wobei

$R^6$ für Alkyl mit 1 bis 4 Kohlenstoffatomen sowie für gegebenenfalls durch Alkyl mit 1 bis 4 Kohlenstoffatomen substituiertes Phenyl steht,

mit Verbindungen der Formel

$$Me-R^2 \qquad (VIII)$$

in welcher

$R^2$ die oben angegebene Bedeutung hat und

Me für ein Alkalimetall, wie vorzugsweise Natrium und Kalium oder Wasserstoff steht,

in Gegenwart eines organischen Lösungsmittels, wie beispielsweise Glykol oder Dimethylformamid, und gegebenenfalls in Gegenwart eines Säurebindemittels, wie beispielsweise Natriumcarbonat, bei Temperaturen zwischen 80 und 150 °C umsetzt.

Die Keto-Derivate der Formel (VII) sind bekannt (vergleiche z. B. DE-OS 2 632 603 und J. Org. Chem. *35,* 2391 (1970)) bzw. können sie in allgemein bekannter Art und Weise erhalten werden.

Die Verbindungen der Formel (VIII) sind allgemein bekannte Verbindungen der organischen Chemie und werden gegebenenfalls in situ eingesetzt.

Die Azole der Formel (IV) und die Alkylierungsmittel der Formel (V) sind ebenfalls allgemein bekannte Verbindungen der organischen Chemie.

Zur Herstellung von physiologisch verträglichen Säureadditionssalzen der Verbindungen der Formel (I) kommen vorzugsweise folgende Säuren infrage : Die Halogenwasserstoffsäuren, z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden :

Dermatomykosen und Systemmykosen durch Trichophyton mentagrophytes und andere Trichophyton-Arten, Mikrosporon-Arten, Epidermophyton floccosum, Sproßpilze und biphasische Pilze sowie Schimmelpilze hervorgerufen.

Als Indikationsgebiet in der Tiermedizin können beispielsweise aufgeführt werden :

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragées, Kapseln, Pillen,

Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragées, Kapseln, Pillen, Granulate, Suppositorien, Lösungen, Suspensionen und Emulsionen, Pasten, Salben, Gele, Cremes, Lotions, Puder und Sprays genannt.

Tabletten, Dragées, Kapseln, Pillen und Granulate können den oder die Wirkstoffe neben den üblichen Trägerstoffen enthalten, wie

a) Füll- und Streckmittel, z. B. Stärken, Milchzucker, Rohrzucker, Glukose, Mannit und Kieselsäure,
b) Bindemittel, z. B. Carboxymethylcellulose, Alginate, Gelatine, Polyvinylpyrrolidon,
c) Feuchthaltemittel, z. B. Glycerin,
d) Sprengmittel, z. B. Agar-Agar, Calciumcarbonat und Natriumbicarbonat,
e) Lösungsverzögerer, z. B. Paraffin und
f) Resorptionsbeschleuniger, z. B. quarternäre Ammoniumverbindungen,
g) Netzmittel, z. B. Cetylalkohol, Glycerinmonostearat,
h) Adsorptionsmittel, z. B. Kaolin und Bentonit und
i) Gleitmittel, z. B. Talkum, Calcium- und Magnesiumstearat und feste Polyethylenglykole oder Gemische der unter (a) bis (i) aufgeführten Stoffe.

Die Tabletten, Dragées, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

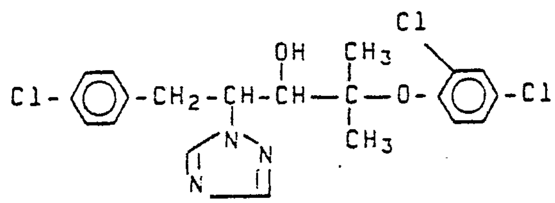

x HCl

10 g (0,022 8 Mol) 5-(4-Chlorphenyl)-2-(2,4-dichlorphenoxy)-2-methyl-4-(1,2,4-triazol-1-yl)-pentan-3-on werden in 100 ml Methanol gelöst, 1 g Natriumborhydrid portionsweise bei 0-5 °C zugegeben, 15 Stunden bei Raumtemperatur nachgerührt und 50 ml 2n Salzsäure zugegeben. Nach 4 Stunden wird durch Abdestillieren des Lösungsmittels im Vakuum eingeengt. Der Rückstand wird in 200 ml Methylenchlorid aufgenommen, die organische Phase mit 100 ml gesättigter Natriumhydrogencarbonatlösung verrührt, die organische Phase abgetrennt, zweimal mit je 100 ml Wasser gewaschen, über Natriumsulfat getrocknet und im Vakuum eingeengt. Der Rückstand wird in 100 ml Ether gelöst und mit etherischer Salzsäure versetzt. Man erhält 7,8 g (81 % der Theorie) 5-(4-Chlorphenyl)-2-(2,4-dichlorphenoxy)-2-methyl-4-(1,2,4-triazol-1-yl)-pentan-3-ol-hydrochlorid vom Schmelzpunkt 46-50 °C.

### Herstellung des Ausgangsproduktes

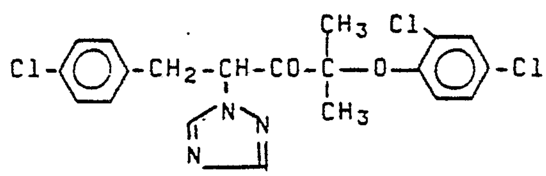

Zu 31,4 g (0,1 Mol) 3-(2,4-Dichlorphenoxy)-3-methyl-1-(1,2,4-triazol-1-yl)-butan-2-on in 100 ml Dimethylsulfoxid werden unter Kühlung bei 20 °C zunächst 5,6 g Kaliumhydroxid in 12 ml Wasser und danach 16,1 g (0,1 Mol) 4-Chlorbenzylchlorid in 5 ml Dimethylsulfoxid getropft. Man läßt 15 Stunden bei 20 °C nachreagieren, gibt die Lösung auf 200 ml Wasser, extrahiert mit 200 ml Methylenchlorid, wäscht die organische Phase dreimal mit je 200 ml Wasser, trocknet über Natriumsulfat und destilliert das Lösungsmittel im Wasserstrahlvakuum ab. Der Rückstand wird in 200 ml Ether aufgenommen, am Rückfluß erhitzt und die ausgefallenen Kristalle abgesaugt. Man erhält 21,3 g (48 % der Theorie) 5-(4-Chlorphenyl)-2-(2,4-dichlorphenoxy)-2-methyl-4-(1,2,4-triazol-1-yl)-pentan-3-on vom Schmelzpunkt 104-108 °C.

### Beispiel 2

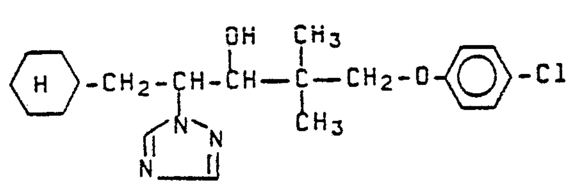

10 g (0,034 Mol) 1-(4-Chlorphenoxy)-5-cyclohexyl-2,2-dimethyl-4-(1,2,4-triazol-1-yl)-pentan-3-on werden in 100 ml Methanol gelöst, 1,7 g Natriumborhydrid bei 0-5 °C portionsweise zugegeben, 15 Stunden bei Raumtemperatur nachgerührt und 20 ml 2n Salzsäure zugetropft. Nach 5 Stunden wird durch Abdestillieren des Lösungsmittels im Vakuum eingeengt, der Rückstand in 100 ml Methylenchlorid aufgenommen, die organische Phase mit 100 ml gesättigter Natriumhydrogencarbonatlösung verrührt, die organische Phase abgetrennt, zweimal mit je 50 ml Wasser gewaschen, über Natriumsulfat getrocknet und im Vakuum eingeengt. Der Rückstand wird mit 50 ml Diisopropylether verrieben. Man erhält 7,2 g (54 % der Theorie) 1-(4-Chlorphenoxy)-5-cyclohexyl-2,2-dimethyl-4-(1,2,4-triazol-1-yl)-pentan-3-ol vom Schmelzpunkt 105-108 °C.

Herstellung des Ausgangsproduktes

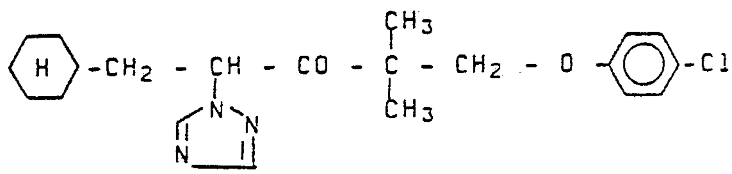

Zu 29,3 g (0,1 Mol) 1-(4-Chlorphenoxy)-2,2-dimethyl-4-(1,2,4-triazol-1-yl)-butan-3-on in 100 ml Dimethylsulfoxid werden bei 20 °C zunächst 5,6 g Kaliumhydroxid in 12 ml Wasser und danach 17,7 g (0,1 Mol) Cyclohexylmethylbromid in 5 ml Dimethylsulfoxid getropft. Man läßt das Reaktionsgemisch 15 Stunden bei Raumtemperatur nachrühren, gibt es auf 200 ml Wasser und extrahiert mit 200 ml Methylenchlorid. Die organische Phase wird dreimal mit je 100 ml Wasser gewaschen, über Natriumsulfat getrocknet und durch Abdestillieren des Lösungsmittels eingeengt. Der Rückstand wird in 100 ml Ether aufgenommen, wobei er auskristallisiert. Man erhält 18,6 g (47 % der Theorie) 1-(4-Chlorphenoxy)-5-cyclohexyl-2,2-dimethyl-4-(1,2,4-triazol-1-yl)-pentan-3-on vom Schmelzpunkt 58-60 °C.

In entsprechender Weise werden die folgenden erfindungsgemäßen Verbindungen der allgemeinen Formel

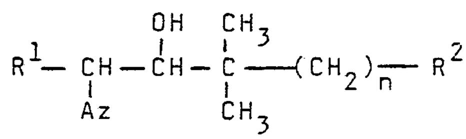 (I)

erhalten :

| Bsp. Nr. | $R^1$ | Az | n | $R^2$ | Schmelzpunkt (°C) oder Brechungsindex $n_D^{20}$ |
|---|---|---|---|---|---|
| 3 | 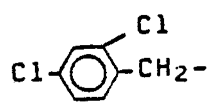 | 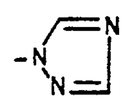 | 1 | $-O-CH_3$ | 120(Zers.) (x HCl) |
| 4 | 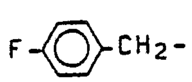 | 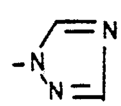 | 1 | $-O-CH_3$ | 86-90 |
| 5 | 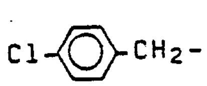 | 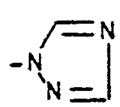 | 1 | $-O-CH_3$ | 104-108 |
| 6 | 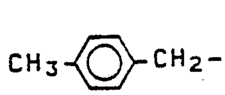 | 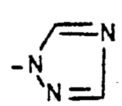 | 1 | 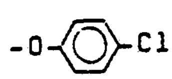 | 158 |
| 7 | 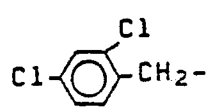 | 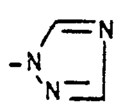 | 1 | 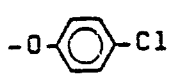 | 128 |
| 8 | 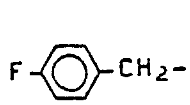 | 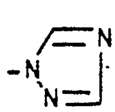 | 1 | 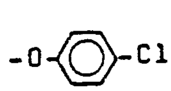 | 167-168 |

(Fortsetzung)

| Bsp. Nr. | $R^1$ | Az | n | $R^2$ | Schmelzpunkt (°C) oder Brechungsindex $n_D^{20}$ |
|---|---|---|---|---|---|
| 9 | $C_4H_9$-n | 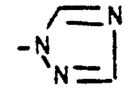 | 1 | 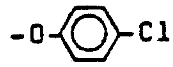 | zähes Oel |
| 10 | 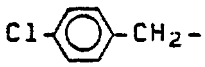 | 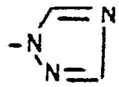 | 0 | $-CO-OC_2H_5$ | 120 |
| 11 | 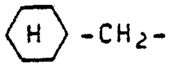 | 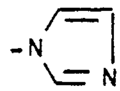 | 1 | 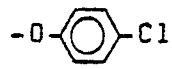 | 67-70 |
| 12 | 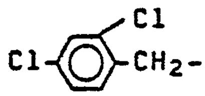 | 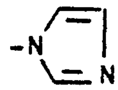 | 1 | 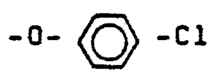 | 164-168 |
| 13 | 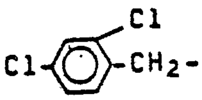 | 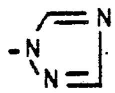 | 0 | 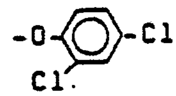 | 192-194 (x 1/2 NDS) |
| 14 | 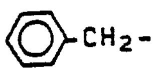 | 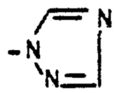 | 0 | 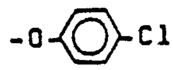 | 178-80 (x 1/2 NDS) |
| 15 | 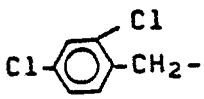 | 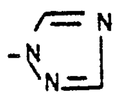 | 0 | 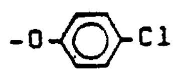 | 45-48 |
| 16 | 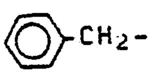 | 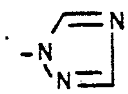 | 0 | 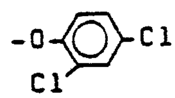 | 204 (x 1/2 NDS) |
| 17 | 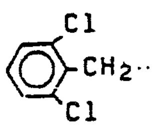 | 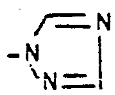 | 0 | 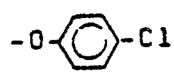 | 150 (x 1/2 NDS) |
| 18 | 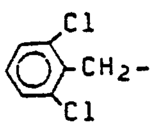 | 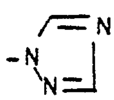 | 0 | 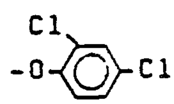 | 200 (x 1/2 NDS) |
| 19 | 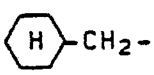 | 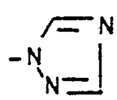 | 0 | 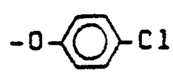 | 124 |
| 20 | 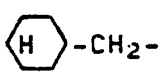 | 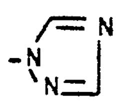 | 0 | 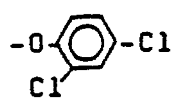 | 176-178 (x 1/2 NDS) |
| 21 | 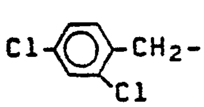 | 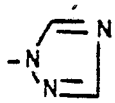 | 1 | 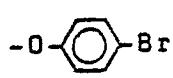 | 140-142 |
| 22 | 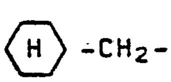 | 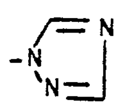 | 1 | 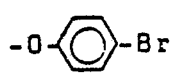 | 148 |

(Fortsetzung)

| Bsp. Nr. | $R^1$ | Az | n | $R^2$ | Schmelzpunkt (°C) oder Brechungsindex $n_D^{20}$ |
|---|---|---|---|---|---|
| 23 | 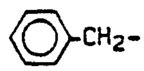 | 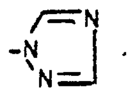 | 1 | 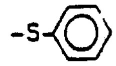 | 78-80 |
| 24 | 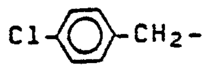 | 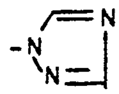 | 1 | 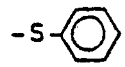 | 25 |
| 25 | 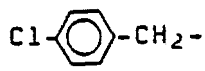 | 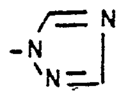 | 1 | 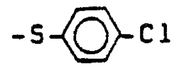 | 124-16 |
| 26 | 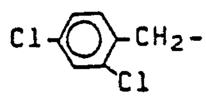 | 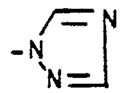 | 1 | 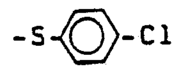 | 48 |
| 27 | 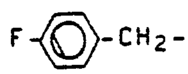 | 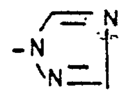 | 1 | 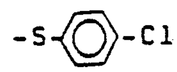 | 152-153 |
| 28 | $C_4H_9$-n | 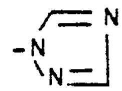 | 1 | 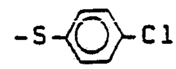 | $n_D^{20}$ 1,5458 (Brechungsindex) |
| 29 | 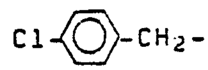 | 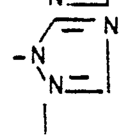 | 1 | $-OC_2H_5$ | 40 |
| 30 | 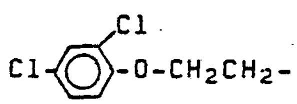 | 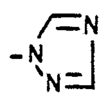 | 1 | 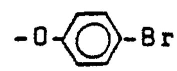 | 69-76 |
| 31 | $C_4H_9$-n | 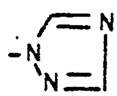 | 1 | 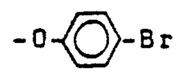 | 69-72 |
| 32 | 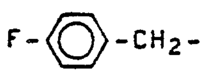 | 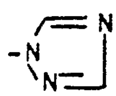 | 1 | 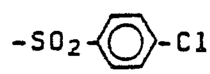 | 40 |
| 33 | 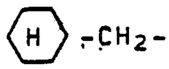 | 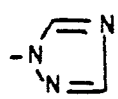 | 1 | $-OC_2H_5$ | 30 |
| 34 | 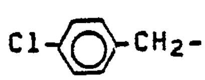 | 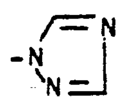 | 1 | 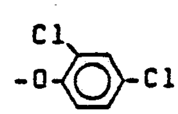 | 145-48 |
| 35 | 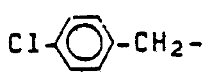 | 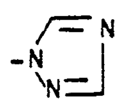 | 1 | 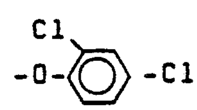 | 148 |
| 36 | $C_4H_9$-n | 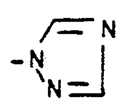 | 0 | 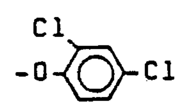 | 1,545 |

(Fortsetzung)

| Bsp. Nr. | $R^1$ | Az | n | $R^2$ | Schmelzpunkt (°C) oder Brechungsindex $n_D^{20}$ |
|---|---|---|---|---|---|
| 37 | $C_4H_9$-n | 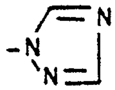 | 0 | 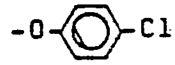 | 1,535 |
| 38 | $CH_2$=CH-$CH_2$- | 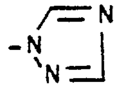 | 0 | 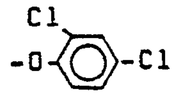 | 1,556 |
| 39 | $C_2H_5$ | 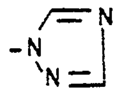 | 0 | 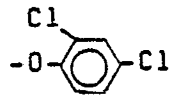 | 110 |
| 40 | $CH_3$ | 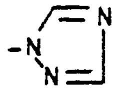 | 0 | 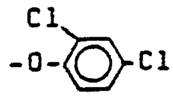 | 145 |
| 41 | $CH_2$=CH-$CH_2$- | 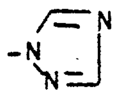 | 0 | 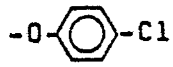 | 1,547 |
| 42 | $C_2H_5$ | 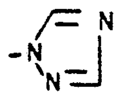 | 0 | 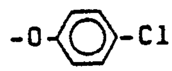 | 1,540 |
| 43 | $CH_3$ | 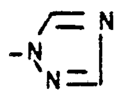 | 0 | 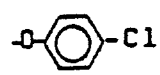 | 1,559 |
| 44 | $C_2H_5$ | 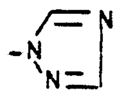 | 0 | 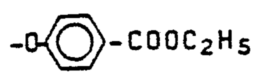 | 1,535 |
| 45 | $CH_2$=CH-$CH_2$- | 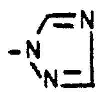 | 0 | 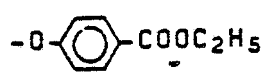 | $n_D^{20}$ 1,521 |
| 46 | $CH_3$ | 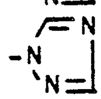 | 0 | 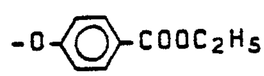 | 84-96 |
| 47 | $C_4H_9$-n | 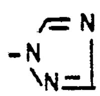 | 0 | 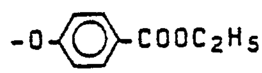 | $n_D^{20}$ 1,535 |
| 48 | 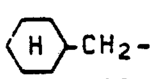 | 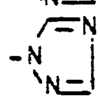 | 0 | 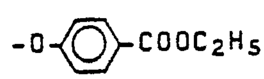 | $n_D^{20}$ 1,534 |
| 49 | 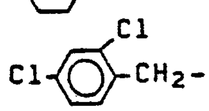 | 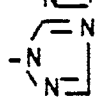 | 0 | 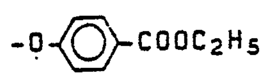 | 104-09 |
| 50 | $CH_2$=CH-$CH_2$- | 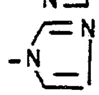 | 0 | 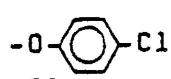 | 141-45 |
| 51 | $CH_2$=CH-$CH_2$- | 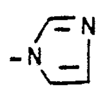 | 0 | 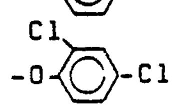 | 100-04 |
| 52 | 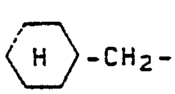 | 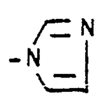 | 0 | 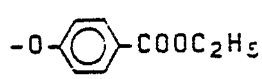 | 136-40 |

(Fortsetzung)

| Bsp. Nr. | $R^1$ | Az | n | $R^2$ | Schmelzpunkt (°C) oder Brechungsindex $n_D^{20}$ |
|---|---|---|---|---|---|
| 53 | $CH_2=CH-CH_2-$ | 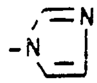 | 0 | 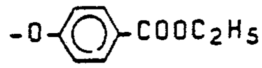 | 106-10 |
| 54 | 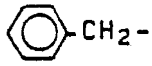 | 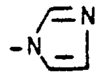 | 0 | 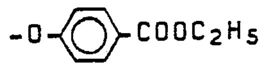 | 130-34 |
| 55 | 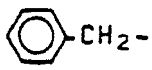 | 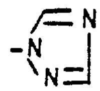 | 1 | 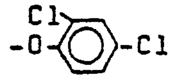 | 155-56 |
| 56 | 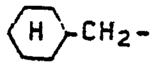 | 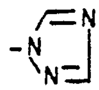 | 1 | 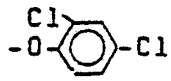 | 145-48 |
| 57 | 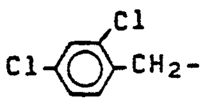 | 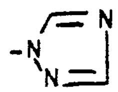 | 1 | 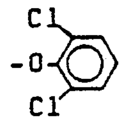 | 114-20 |
| 58 | 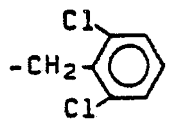 | 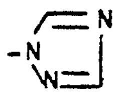 | 1 | 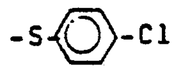 | 168 |
| 59 | 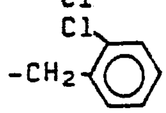 | 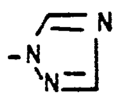 | 1 | 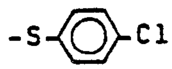 | ca.30 |
| 60 | $C_4H_9$-n | 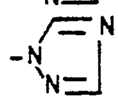 | 1 | 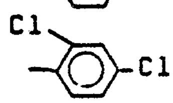 | 106 |
| 61 | $C_4H_9$-n | 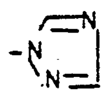 | 1 | 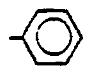 | 105 |
| 62 | 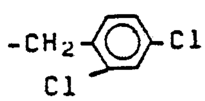 | 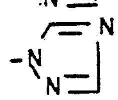 | 1 | 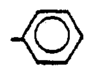 | 178 |
| 63 | $C_4H_9$-n | 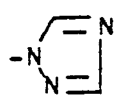 | 1 | 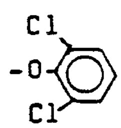 | 160-2 (x HCl) |
| 64 | 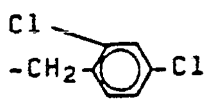 | 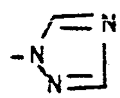 | 1 | $-O-C_2H_5$ | 155-59 (x HCl) |
| 65 | 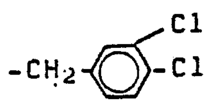 | 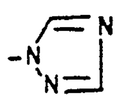 | 1 | $-O-C_2H_5$ | 148-50 (x HCl) |
| 66 | 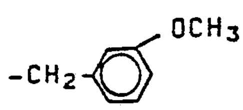 | 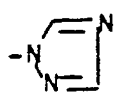 | 1 | 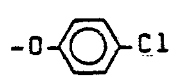 | 127-30 (x HCl) |
| 67 | 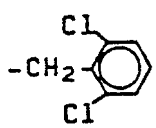 | 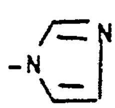 | 0 | 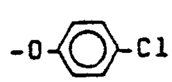 | 180-81 |

(Fortsetzung)

| Bsp. Nr. | $R^1$ | Az | n | $R^2$ | Schmelzpunkt (°C) oder Brechungsindex $n_D^{20}$ |
|---|---|---|---|---|---|
| 68 | $C_4H_9$-n | 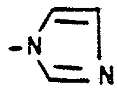 | 0 | 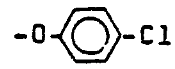 | 118-120 |
| 69 | $C_4H_9$-n | 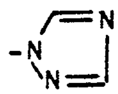 | 0 | 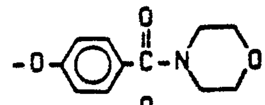 | Harz |
| 70 | $-CH_2-CH=CH_2$ | 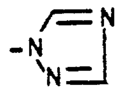 | 0 | 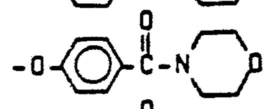 | Harz |
| 71 | 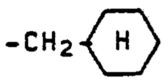 | 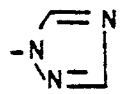 | 0 | 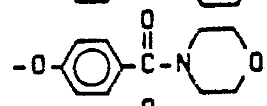 | Harz |
| 72 | 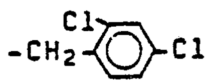 | 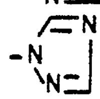 | 0 | 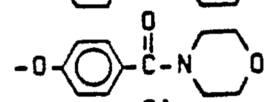 | 158-164 |
| 73 | $-CH_2-C\equiv CH$ | 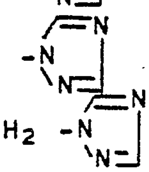 | 1 | 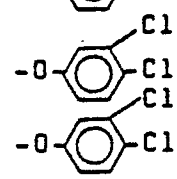 | 156-58 (xHCl) |
| 74 | $-CH_2CH_2-CH=CH_2$ | | 1 | | 115-25 (x HCl) |
| 75 | 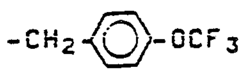 | 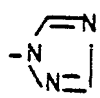 | 1 | 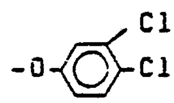 | 142-45 |
| 76 | $-C_2H_5$ | 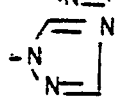 | 1 | 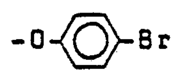 | 148-54 (x HCl) |
| 77 | $C_4H_9$-n | 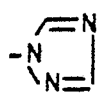 | 1 | 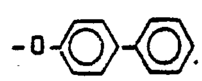 | 154-74 (x HCl) |
| 78 | $C_4H_9$-n | 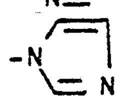 | 1 | 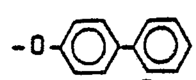 | Oel (x HCl) |
| 79 | $C_2H_5$ | 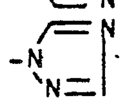 | 0 | 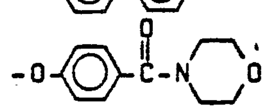 | 168-72 (x 1/2 NDS) |
| 80 | $C_2H_5$ | 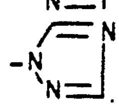 | 0 | 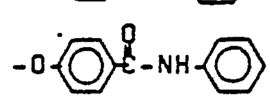 | 133-143 |
| 81 | $C_2H_5$ | 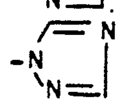 | 0 | 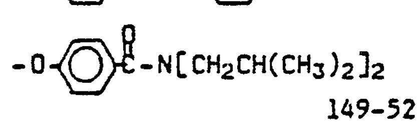 | 149-52 (x 1/2 NDS) |
| 82 | 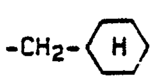 | 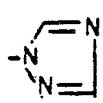 | 0 | 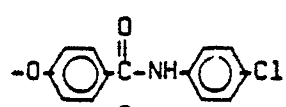 | 133-38 |
| 83 | 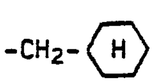 | 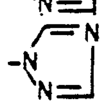 | 0 | 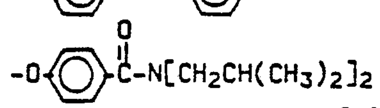 | Oel |
| 84 | 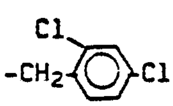 | 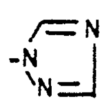 | 1 | 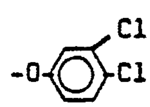 | 132-40 |

(Fortsetzung)

| Bsp. Nr. | $R^1$ | Az | n | $R^2$ | Schmelzpunkt (°C) oder Brechungsindex $n_D^{20}$ |
|---|---|---|---|---|---|
| 85 | $C_4H_9$-n | 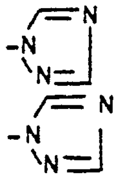 | 1 | 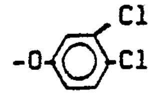 | 138-41 (x HCl) |
| 86 | 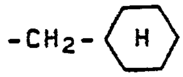 | | 1 | 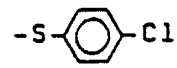 | $n_D^{20}$ 1,5624 |
| 87 | $CH_2$=CH-$CH_2$- | 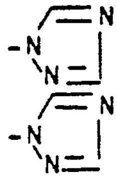 | 1 | 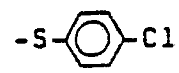 | $n_D^{20}$ 1,5739 |
| 88 | CH≡C-$CH_2$- | | 1 | 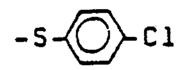 | 90-92 |
| 89 | 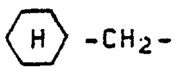 | 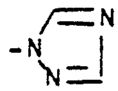 | 1 | 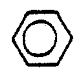 | 138 |
| 90 | 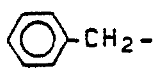 | 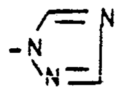 | 1 | 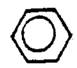 | 99 |
| 91 | 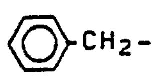 | 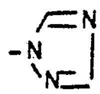 | 1 | 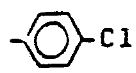 | 161-62 |
| 92 | 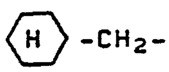 | 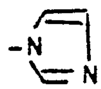 | 1 | 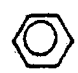 | Oel |
| 93 | $C_4H_9$-n | 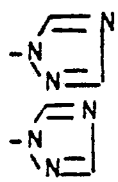 | 0 | 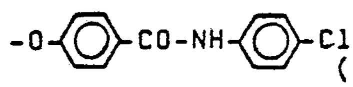 | 164-65 (x1/2 NDS) |
| 94 | $CH_2$=CH-$CH_2$- | | 0 | 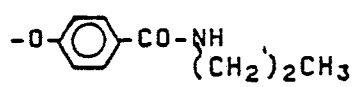 | 157-62 (x1/2 NDS) |
| 95 | $CH_2$=CH-$CH_2$- | 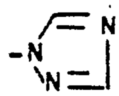 | 0 | 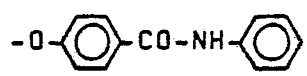 | 128-34 |
| 96 | $C_4H_9$-n | 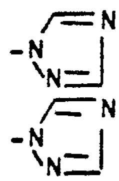 | 1 | 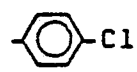 | 92-93 |
| 97 | 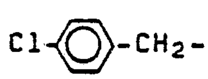 | | 1 | 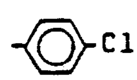 | 110 |
| 98 | 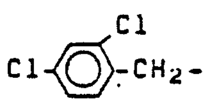 | 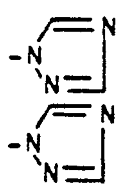 | 1 | 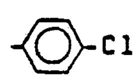 | 130-31 |
| 99 | 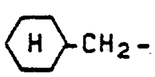 | | 1 | 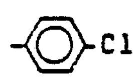 | 189 |
| 100 | 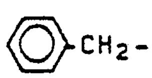 | 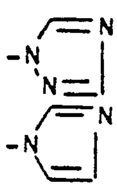 | 1 | 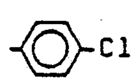 | 117 |
| 101 | $C_4H_9$-n | | 1 | 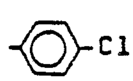 | 121 |
| 102 | 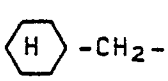 | 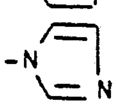 | 1 | 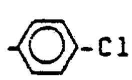 | 160 |

(Fortsetzung)

| Bsp. Nr. | $R^1$ | Az | n | $R^2$ | Schmelzpunkt (°C) oder Brechungsindex $n_D^{20}$ |
|---|---|---|---|---|---|
| 103 | 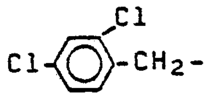 | 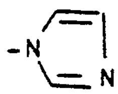 | 1 | 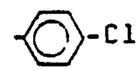 | 155-57 |
| 104 | 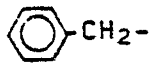 | 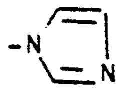 | 1 | 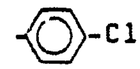 | 74-76 |
| 105 | 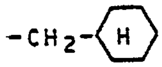 | 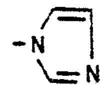 | 1 | 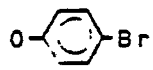 | 65-70 |
| 106 | 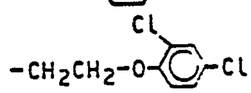 | 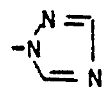 | 1 | 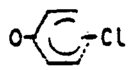 | 145-155 (x HCl) |
| 107 | 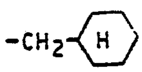 | 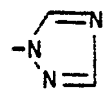 | 1 | $-OCH_3$ | 60-65 |
| 108 | $-CH_2CH_2-CH=CH_2$ | 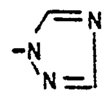 | 1 | 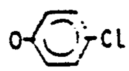 | 144-46 (xHCl) |
| 109 | $-CH_2(CH_2)_4-CH_3$ | 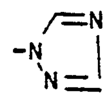 | 1 | 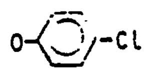 | 158-60 (xHCl) |
| 110 | $-CH_2CH_2-CH_3$ | 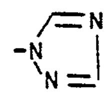 | 1 | 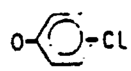 | 150-52 (xHCl) |
| 111 | $-CH_2-CH=CH_2$ | 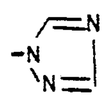 | 1 | 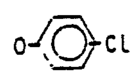 | 164-66 (x HCl) |
| 112 | $-CH_2-CH=CH_2$ | 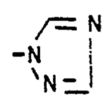 | 1 | 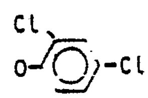 | 122-14 |
| 113 | $-CH_2CH_2-CH=CH_2$ | 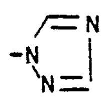 | 1 | 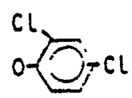 | 1,544 |
| 114 | $-CH_2-(CH_2)_4-CH_3$ | 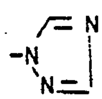 | 1 | 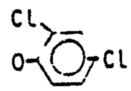 | 148-50 (x HCl) |
| 115 | $-CH_2-CH_3$ | 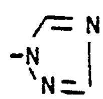 | 1 | 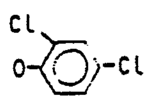 | 134-38 (x HCl) |
| 116 | $-CH_2CH_2-CH_3$ | 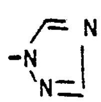 | 1 | 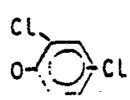 | 100-130 (x HCl) |
| 117 | $-CH_2CH_2CH_2-CH_3$ | 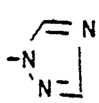 | 1 | 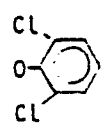 | 145-48 (x HCl) |

NDS = 1,5-Naphthalindisulfonsäure

Verwendungsbeispiele

In den nachfolgenden Beispielen werden die nachstehend angegebenen Verbindungen als Vergleichssubstanzen eingesetzt :

(A) 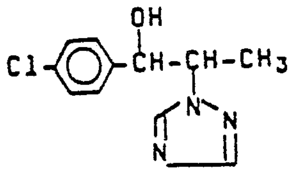

(B) 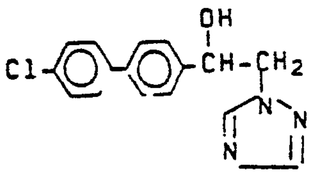

Beispiel A

Antimykotische in-vitro-Wirksamkeit

Versuchsbeschreibung :
Die in-vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze :
Sabourand's milieu d'épreuve

b) für Hefen :
Fleischextrakt-Traubenzucker-Bouillon.

Die Bebrütungstemperatur betrug 20 °C, die Bebrütungsdauer lag bei 24 bis 96 Stunden bei Hefen und 96 Stunden bei Dermatophyten und Schimmelpilzen.
In diesem Test zeigen insbesondere die Verbindungen der Beispiele 1, 2, 3, 5, 6, 7, 11, 13, 14, 15, 16, 17, 19, 22, 24, 25, 26, 28, 29, 30, 31, 36, 37, 38, 41, 42, 47, 49, 51, 60, 62, 64, 65, 66, 74, 77 und 104 eine bessere antimykotische Wirkung als die aus dem Stand der Technik bekannten Verbindungen (A) und (B).

Tabelle A
Antimykotische in-vitro-Wirksamkeit

| Wirkstoff | MHK-Werte in $\gamma$/ml Nährmedium Trichophyton mentagr. | Candida albicans | Aspergillus fumig | Microsporum canis | Torulopsis glabrata |
|---|---|---|---|---|---|
| (A) (bekannt) | > 64 | > 64 | — | — | — |
| (B) (bekannt) | 4 | > 64 | > 64 | > 64 | — |
| gemäß Herst. Bsp. | | | | | |
| 1 | 2 | 16 | 64 | 64 | 64 |
| 2 | < 1 | < 1 | 2 | 2 | < 1 |
| 3 | 8 | 8 | 64 | 8 | 16 |
| 5 | 4 | 8 | 16 | 4 | 32 |
| 6 | < 1 | < 1 | 4 | < 1 | < 1 |
| 7 | < 1 | < 1 | 64 | < 1 | < 1 |
| 11 | < 1 | < 1 | 4 | 4 | 8 |
| 13 | < 1 | > 64 | > 64 | 8 | 64 |
| 14 | < 1 | < 1 | 8 | < 1 | 4 |
| 15 | < 1 | < 1 | > 64 | 4 | < 1 |
| 16 | 2 | 16 | 16 | 4 | 64 |
| 17 | < 1 | < 1 | > 64 | 4 | 4 |

Tabelle A (Fortsetzung)
Antimykotische in-vitro-Wirksamkeit

| Wirkstoff | MHK-Werte in γ/ml Nährmedium<br>Trichophyton mentagr. | Candida albicans | Aspergillus fumig | Microsporum canis | Torulopsis glabrata |
|---|---|---|---|---|---|
| 19 | 2 | < 1 | 8 | 32 | 4 |
| 22 | < 1 | 32 | < 1 | 8 | < 1 |
| 24 | < 1 | 4 | 64 | 4 | 2 |
| 25 | < 1 | < 1 | 64 | < 1 | < 1 |
| 26 | < 1 | < 1 | 64 | 16 | < 1 |
| 27 | < 1 | < 1 | < 1 | < 1 | 4 |
| 28 | < 1 | < 1 | > 64 | < 1 | 2 |
| 29 | < 1 | 2 | 8 | 8 | 16 |
| 30 | < 1 | < 1 | 32 | 8 | 4 |
| 31 | < 1 | < 1 | 4 | < 1 | < 1 |
| 36 | < 1 | 2 | 8 | 2 | 8 |
| 37 | 2 | < 1 | 16 | 2 | 4 |
| 38 | 2 | 4 | 8 | 2 | 32 |
| 41 | 8 | 4 | 8 | 4 | 8 |
| 42 | 8 | 8 | 32 | 8 | 32 |
| 47 | 2 | 8 | 8 | 8 | 32 |
| 49 | 4 | < 1 | 64 | 2 | 64 |
| 51 | 4 | < 1 | 16 | 8 | 4 |
| 60 | < 1 | 2 | < 1 | 2 | 8 |
| 62 | < 1 | < 1 | 32 | 8 | 4 |
| 64 | 4 | < 1 | 16 | 8 | 4 |
| 65 | 2 | 16 | 8 | 4 | 32 |
| 66 | < 1 | < 1 | 16 | 8 | 2 |
| 74 | < 1 | 2 | 8 | < 1 | 32 |
| 77 | < 1 | 8 | 4 | 8 | 32 |
| 102 | < 1 | < 1 | 8 | 16 | 8 |

Beispiel B

Antimykotische in-vivo-Wirksamkeit (oral) bei Mäusecandidose

Versuchsbeschreibung

Mäuse vom Typ SPF-$CF_1$ wurden intravenös mit 1-2 × $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 50-100 mg/kg Körpergewicht der Präparate oral behandelt.

Ergebnis

Unbehandelte Tiere starben 3 bis 6 Tage post infektionem. Die Überlebensrate am 6. Tag post infektionem betrug bei unbehandelten Kontrolltieren etwa 5 %.

In diesem Test zeigen z. B. die erfindungsgemäßen Verbindungen 7, 11, 31, 66, 77 und 104 eine bessere Wirkung als die aus dem Stand der Technik bekannten Verbindungen (A) und (B).

Zeichenerklärung :

+++++ = sehr gute Wirkung = 90 % Überlebende am 6.Tag p. i.
++++ = gute Wirkung = 80 % Überlebende am 6.Tag p. i.
+++ = Wirkung = 60 % Überlebende am 6.Tag p. i.
++ = schwache Wirkung = 40 % Überlebende am 6.Tag p. i.
+ = Spur Wirkung = unter 40 % Überlebende am 6.Tag

k. W. = keine Wirkung

Tabelle B

Antimykotische in-vivo-Wirksamkeit (oral) bei Mäusecandidose

| Wirkstoff | Wirkung |
|---|---|
| (A) (bekannt) | k. W. |
| (B) (bekannt) | k. W. |
| gemäß Herst. Bsp. Nr. | |
| 7 | ++ |
| 11 | ++++ |
| 31 | ++++ |
| 66 | ++ |
| 77 | +++ |
| 104 | ++++ |

Beispiel C

Antimikrobielle in-vivo-Wirksamkeit (lokal) am Modell der experimentellen Meerschweinchen-Trichophytie

Versuchsbeschreibung :

Weiße Mäuse der Rasse Pirbright-white wurden auf dem geschorenen, nicht skarifizierten Rücken mit einer Mikro- und Makrokonidien-Suspension von Trichophyton von mentagrophytes infiziert.

Die infizierten Tieren wurden, beginnend mit dem 3. Tag p. i., 1 x täglich mit einer 15 %-igen Lösung der erfindungsgemäßen Präparate (in Dimethylsulfoxid : Glycerin = 1 : 4) lokal behandelt.

Ergebnis :

Bei unbehandelten Tieren entwickelte sich innerhalb 12 Tagen p. i. das typische Bild einer Dermatophytose mit Rötung, Schuppung und Haarausfall bis zum totalen Integument-Defekt an der Infektionsstelle.

In diesem Test zeigen z. B. die erfindungsgemäßen Verbindungen 7, 11 und 31 gute bis sehr gute Wirkung.

Zeichenerklärung :

+++++ = sehr gute Wirkung = keine Infektionszeichen am 12.-15. Tag p. i.
++++ = gute Wirkung = geringe Rötung, vereinzelt Schuppen
+++ = Wirkung = Rötung, Schuppung, ohne Haarausfall
++ = schwache Wirkung = Rötung, Schuppung, Haarausfall
+ = Spur Wirkung = flächiger Haarausfall, entzündliche Hautreaktion

Tabelle C

Antimykotische in-vivo-Wirksamkeit (lokal) am Modell der experimentellen Meerschweinchen-Trichophytie

| Wirkstoff | Wirkung |
|---|---|
| gem. Herst. Bsp. Nr. | |
| 7 | +++++ |
| 11 | ++++ |
| 31 | ++++ |

Beispiel D/Formulierungen

1. Lösung

| | |
|---|---|
| Wirkstoff gemäß Formel (I) : | 10 g |
| Alkohol, rein (96 %-ig) : | 300 g |
| Isopropylmyristat : | 526 g |
| | 836 g |

2. Creme

| | |
|---|---|
| Wirkstoff gemäß Formel (I) : | 10 g |
| Arlacel 60® : (Sorbitan-monostearat) | 20 g |
| Tween 60® : (Polyoxyethylen(20)-sorbitan-monostearat) | 15 g |
| Walrat, künstlich : (Mischung von Estern von gesättigten Fettsäuren $C_{14}$-$C_{18}$ und Fettalkoholen $C_{14}$-$C_{18}$) | 30 g |
| Lanette O® : (Gemisch aus Cetyl-Alkohol und Stearyl-Alkohol) | 100 g |
| Entanol G® : (2-Octyl-dodecanol) | 135 g |
| Benzylalkohol : | 10 g |
| Wasser, entmineralisiert : | 680 g |
| | 1 000 g |

**Patentansprüche**

1. 1-Azolyl-butan-2-ole der Formel

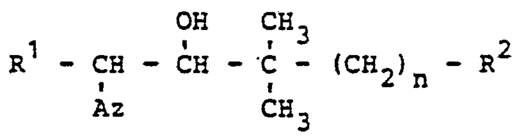

(I)

in welcher

Az für 1,2,4-Triazol-1-yl und -4-yl oder Imidazol-1-yl steht,

n für 0 oder 1 steht,

$R^1$ für geradkettiges oder verzweigtes Alkyl mit 1 bis 6 Kohlenstoffatomen, Alkenyl und Alkinyl mit jeweils 2 bis 6 Kohlenstoffatomen, gegebenenfalls durch Methyl substituiertes Cyclohexyl und Cyclohexylmethyl, sowie für gegebenenfalls substituiertes Phenoxyalkyl und gegebenenfalls substituiertes Phenylalkyl mit jeweils 1 bis 2 Kohlenstoffatomen im Alkylteil steht, wobei als Phenyl-Substituenten genannt seien ; Fluor, Chlor, Methyl, Ethyl, Isopropyl, tert.-Butyl, Dimethylamino, Methoxy, Methylthio, Cyclohexyl, Trifluormethyl, Trifluormethoxy, Trifluormethylthio, Nitro, Cyano sowie gegebenenfalls durch Fluor und Chlor substituiertes Phenyl und Phenoxy, sowie die Morpholinocarbinol-, Phenylaminocarbonyl-, Chlorphenylaminocarbonyl- und Dibutylaminocarbonyl-Gruppe,

$R^2$ für gegebenenfalls substituiertes Phenyl steht, wobei als Substituenten die bei $R^1$ bereits genannten Phenylsubstituenten infrage kommen, ferner für Cyano, Methoxycarbonyl, Ethoxycarbonyl, Isopropoxycarbonyl, sowie die Gruppierungen —X—$R^3$ und —CO—$NR^4R^5$ steht, in welchen

X für Sauerstoff, Schwefel, die SO- oder $SO_2$-Gruppe steht,

$R^3$ für geradkettiges oder verzweigtes Alkyl mit 1 bis 4 Kohlenstoffatomen oder gegebenenfalls substituiertes Phenyl und Benzyl steht, wobei als Substituenten die bei $R^1$ bereits genannten Phenylsubstituenten infrage kommen,

$R^4$ für Wasserstoff, Methyl, Ethyl, Isopropyl oder gegebenenfalls substituiertes Phenyl steht, wobei als Substituenten Halogen und Alkyl mit 1 bis 4 Kohlenstoffatomen infrage kommen, und

$R^5$ für Wasserstoff, Methyl oder Isopropyl steht,

sowie deren Säureadditionssalzen zur Verwendung als Antimykotikum.

2. 5-(4-Chlorphenyl)-2-(2,4-dichlorphenoxy)-2-methyl-4-(1,2,4-triazol-1-yl)-pentan-3-ol zur Verwendung als Antimykotikum.

3. 1-(4-Chlorphenoxy)-5-cyclohexyl-2,2-dimethyl-4-(1,2,4-triazol-1-yl)-pentan-3-ol zur Verwendung als Antimykotikum.

4. 4-(4-Chlorphenylmercapto)-3,3-dimethyl-1-(imidazol-1-yl)-butan-2-ol zur Verwendung als Antimykotikum.

5. Verfahren zur Herstellung eines antimykotischen Mittels, dadurch gekennzeichnet, daß man substituierte 1-Azolylbutan-2-ole gemäß der Formel in Anspruch 1 mit inerten, nichttoxischen, pharmazeutisch geeigneten Trägerstoffen vermischt.

**Claims**

1. Azol-1-yl-butan-2-ols of the formula

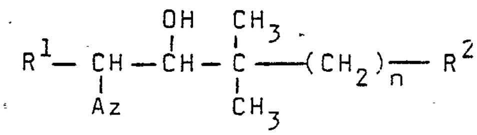 (I)

in which

Az represents 1,2,4-triazol-1-yl or -4-yl or imidazol-1-yl,

n represents 0 or 1,

$R^1$ represents straight-chain or branched alkyl with 1 to 6 carbon atoms, alkenyl or alkinyl with in each case 2 to 6 carbon atoms, optionally methyl-substituted cyclohexyl or cyclohexylmethyl, or optionally substituted phenoxyalkyl or optionally substituted phenylalkyl with in each case 1 to 2 carbon atoms in the alkyl part, the following being mentioned as phenyl substituents ; fluorine, chlorine, methyl, ethyl, isopropyl, tert.-butyl, dimethylamino, methoxy, methylthio, cyclohexyl, trifluoromethyl, trifluoromethoxy, trifluoromethylthio, nitro, cyano and optionally fluorine- and chlorine-substituted phenyl and phenoxy, and the morpholinocarbinol, phenylaminocarbonyl, chlorophenylaminocarbonyl and dibutylaminocarbonyl group,

$R^2$ represents optionally substituted phenyl, suitable substituents being the phenyl substituents already mentioned under $R^1$, or cyano, methoxycarbonyl, ethoxycarbonyl, isopropoxycarbonyl, or the groupings —X—$R^3$ or —CO—$NR^4R^5$, in which

X represents oxygen, sulphur or the SO or $SO_2$ group, $R^3$ represents straight-chain or branched alkyl with 1 to 4 carbon atoms or optionally substituted phenyl or benzyl, suitable substituents being those already mentioned under $R^1$,

$R^4$ represents hydrogen, methyl, ethyl, isopropyl or optionally substituted phenyl, suitable substituents being halogen or alkyl with 1 to 4 carbon atoms, and

$R^5$ represents hydrogen, methyl or isopropyl,

and acid addition salts thereof for use as an antimycotic.

2. 5-(4-Chlorophenyl)-2-(2,4-dichlorophenoxy)-2-methyl-4-(1,2,4-triazol-1-yl)-pentan-3-ol for use as an antimycotic.

3. 1-(4-Chlorophenoxy)-5-cyclohexyl-2,2-dimethyl-4-(1,2,4-triazol-1-yl)-pentan-3-ol for use as an antimycotic.

4. 4-(4-Chlorophenylmercapto)-3,3-dimethyl-1-(imidazol-1-yl)-butan-2-ol for use as an antimycotic.

5. Process for the preparation of an antimycotic agent, characterised in that substituted azol-1-yl-butan-2-ols according to the formula in Claim 1 are mixed with inert, non-toxic, pharmaceutically suitable excipients.

**Revendications**

1. 1-azolyl-butan-2-ols de formule

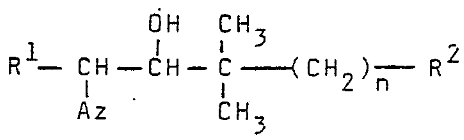 (I)

dans laquelle

Az représente un groupe 1,2,4-triazol-1-yl, un groupe 1,2,4-triazol-4-yle ou un groupe imidazol-1-yle,

n est égal à 0 ou 1,

$R^1$ représente un groupe alkyle à chaîne droite ou ramifiée contenant 1 à 6 atomes de carbone, un groupe alcényle et un groupe alcynyle contenant chacun 2 à 6 atomes de carbone, un groupe cyclohexylméthyle ou un groupe cyclohexyle éventuellement substitué par un groupe méthyle, ainsi qu'un groupe phénoxyalkyle éventuellement substitué et un groupe phénylalkyle éventuellement substitué contenant chacun 1 ou 2 atomes de carbone dans la fraction alkyle en mentionnant, comme substituants du groupe phényle : l'atome de fluor, l'atome de chlore, le groupe méthyle, le groupe éthyle, le groupe isopropyle, le groupe tert-butyle, le groupe diméthylamino, le groupe méthoxy, le groupe méthylthio, le groupe cyclohexyle, le groupe trifluorométhyle, le groupe trifluorométhoxy, le groupe trifluorométhylthio, le groupe nitro, le groupe cyano, ainsi que le groupe phénoxy et le groupe phényle éventuellement substitué par un atome de fluor et un atome de chlore, de même que le groupe morpholinocarbinol, le groupe phénylaminocarbonyle, le groupe chlorophénylaminocarbonyle et le groupe dibutylaminocarbonyle,

$R^2$ représente un groupe phényle éventuellement substitué en envisageant, comme substituants, les substituants du groupe phényle déjà mentionnés pour $R^1$, de même qu'un groupe cyano, un groupe méthoxycarbonyle, un groupe éthoxycarbonyle, un groupe isopropoxycarbonyle et les groupements —X—$R^3$ et —CO—$NR^4R^5$ dans lesquels

X représente l'oxygène, le soufre, le groupe SO— ou $SO_2$—,

$R^3$ représente un groupe alkyle à chaîne droite ou ramifiée contenant 1 à 4 atomes de carbone ou un groupe benzyle et un groupe phényle éventuellement substitué en envisageant, comme substituants, les substituants du groupe phényle déjà mentionnés pour $R^1$,

$R^4$ représente un atome d'hydrogène, un groupe méthyle, un groupe éthyle, un groupe isopropyle ou un groupe phényle éventuellement substitué en envisageant, comme substituants, les atomes d'halogènes et les groupes alkyle contenant 1 à 4 atomes de carbone, et

$R^5$ représente un atome d'hydrogène, un groupe méthyle ou un groupe isopropyle,

ainsi que leurs sels d'addition d'acide en vue de les utiliser comme antimycotiques.

2. Le 5-(4-chlorophényl)-2-(2,4-dichlorophénoxy)-2-méthyl-4-(1,2,4-triazol-1-yl)-pentan-3-ol destiné à être utilisé comme antimycotique.

3. Le 1-(4-chlorophénoxy)-5-cyclohexyl-2,2-diméthyl-4-(1,2,4-triazol-1-yl)-pentan-3-ol destiné à être utilisé comme antimycotique.

4. Le 4-(4-chlorophénylmercapto)-3,3-diméthyl-1-(imidazol-1-yl)-butan-2-ol destiné à être utilisé comme antimycotique.

5. Procédé de préparation d'un agent antimycotique, caractérisé en ce qu'on mélange des 1-azolyl-butan-2-ols substitués répondant à la formule de la revendication 1 avec des substances supports inertes, non toxiques et pharmaceutiquement appropriées.